# EUROPEAN PATENT APPLICATION

(11) **EP 1 967 135 A2**
(43) Date of publication of application: **10.09.2008**
(21) Application number: 08001419.4
(22) Date of filing: 25.01.2008
(51) Int. Cl.: A61B 5/05, A61B 5/103

(54) **Stimulation-current providing device and method of controlling stimulation-current providing device**

(30) Priority: 06.03.2007 JP 2007055634; 15.10.2007 WO PCT/JP2007/070041
(71) Applicant: Osachi Co., Ltd., Okaya-shi Nagano 3940085 (JP)
(72) Inventor: Shimazu, Hideaki, Tokyo 154-0021 (JP); Komatsu, Masaru, Okaya-shi, Nagano 394-0085 (JP); Yaguchi, Yasuyuki, Okaya-shi, Nagano 394-0085 (JP)
(74) Representative: Strobel, Wolfgang

(57) **Abstract**

A stimulation-current providing device 1 comprises; an electrode pair 3 including a pair of positive and negative electrodes 3a and 3b and installed on a measured person; and voltage application units 10 to 12 that apply a voltage to the electrode pair 3 so as to apply a stimulation current to the measured person from the electrode pair 3. The voltage application units 10 to 12 apply a pulse- like voltage having a predetermined switching period to the electrode pair 3, while gradually extending or shortening the switching period.

## Description

### Technical Field

The present invention relates to a stimulation-current providing device that provides a stimulation current for a measured person under various tests and a method of controlling the stimulation-current providing device.

### Background of Technology

The present inventor has suggested various pain-measurement devices for objectively measuring levels of pains felt by a measured person (see the patent documents 1 and 2.) These patent documents 1and 2 disclose pain-measurement devices that provides a gradually increased current for a measured person from an electrode, which is installed on such person. Further, the device quantitatively evaluates the levels of pains felt by a measured person, based on the stimulation current given to the person. Namely, the pain-measurement devices, disclosed in the patent documents 1 and 2, measure an amount of electric stimulation( a current value of the stimulation current) corresponding to the strength of pains felt by the measured person, quantitatively evaluating the level of pains felt by the person.

Further, as an electrode installed on the person, the pain -measurement device disclosed in the patent document 1 includes a pair of positive and negative electrodes (an electrode pair) concentrically arranged each other and the pain-measurement device disclosed in the patent document 2 includes a pair of positive and negative electrodes (an electrode pair) adjacently located each other.

Patent document 1 Japanese Patent 3699258

Patent document 2 Japanese Patent 3808492

### Disclosure of the Invention

### Problem to Be Solved

The pain-measurement devices disclosed in the patent documents 1 and 2 can implement various inspections for people with diabetes having nerve damage such as an impairment of a peripheral nerve function for example. The patent documents 1 and 2, however, do not disclose a concrete means for inspecting a neural damage. Further, the pain-measurement device disclosed in the patent document 1 uses a pair of positive and negative electrodes (an electrode pair) concentrically arranged each other and the pain-measurement device disclosed in the patent document 2 uses a pair of positive and negative electrodes adjacently located each other. Hence, the devices in the patent documents 1 and 2 have a disadvantage in that it takes long time to complete various inspections.

One advantage of the present invention is to provide a stimulation-current providing device that is able to appropriately inspect a nerve damage and a method of controlling the stimulation-current providing device and a method of controlling the stimulation-current providing device. Further, the other advantage of the invention is to provide a stimulation-current providing device that is able to shorten inspection- time and a method of controlling the stimulation-current providing device.

### Means to Solve the Problem

In order to overcome the above issue, according to an aspect of the invention, a stimulation-current providing device comprises: an electrode pair including a pair of positive and negative electrodes installed on a measured person; and a voltage application unit that applies a voltage the electrode pair so as to apply a stimulation current to the measured person from the electrode pair. The voltage application unit applies a pulse- like voltage having a predetermined switching period to the electrode pair and applies the stimulation current to the measured person while gradually extending or shortening the switching period.

The stimulation-current providing device in the aspect of the invention is provided with a voltage application unit that applies a pulse- like voltage having a predetermined switching period to the electrode pair, while gradually extending or shortening the switching period. Hence, in the aspect of the invention, it is possible to inspect continuous stimulation felt by a measured person about stimulation from the electrode pair within a specific range in the switching period and oscillating (intermittent) stimulation felt by a measured person about stimulation from the electrode pair within other specific range in the switching period. Namely, the invention is able to obtain inspected values corresponding to neural transfer speeds. Hence, the invention is able to appropriately inspect nerve damage.

Other aspect of the invention is a method of controlling a stimulation-current providing device that includes an electrode pair, composed of a pair of positive and negative electrodes, and provides a stimulation current for a measured person by applying a voltage to the electrode pair. The method comprises: measuring a minimum- perception current value by applying a pulse -like voltage to the electrode pair, the pulse -like voltage having a predetermined initial switching period in order to gradually increase the stimulation current, and the minimum- perception current value being a current value of the stimulation current at the time when the measured person feels initial stimulation (a step of a threshold value); and applying voltage to the electrode pair while gradually extending or shortening the switching period, compared to an initial switching period (a voltage application step.)

The stimulation-current providing device in the other aspect of the invention applies a pulse- like voltage to the electrode pair in the voltage application step, while gradually extending or shortening the switching period, compared to an initial switching period. Hence, it is possible to inspect continuous stimulation felt by the measured person about stimulation from the pair electrode within a specific range in the switching period and oscillating stimulation felt by the measured person about stimulation from the electrode pair within other specific range of the switching period. Namely, the invention is able to obtain inspected values corresponding to neural transfer speeds. Hence, the invention is able to appropriately inspect a neural damage.

In order to overcome the above issue, according to yet other aspect of the invention, a stimulation-current providing device comprises: an electrode sheet that includes a plurality of electrode pairs and is installed on the measured person, each of the electrode pairs being composed of a pair of negative and positive electrodes: a voltage application unit that applies a voltage the electrode pairs so as to apply a stimulation current to the measured person from the electrode pairs. The voltage application unit applies a pulse-like voltage having a predetermined switching period to the plurality of electrode pairs, while shifting timing (on time) of raising the voltage applied to the plurality of electrode pairs each other. Further the voltage application unit applies the voltage to the plurality of electrode pairs, while gradually extending or shortening a switching period so as to provide the stimulation current for the measured person.

The stimulation-current providing device in the yet other aspect of the invention is provided with a voltage application unit that applies a pulse- like voltage having a predetermined switching period to the electrode pairs, while shifting the "on time" of applying the voltage to the plurality of electrode pairs each other and gradually extending or shortening the switching period. Hence, it is possible to inspect one point of stimulation felt by the measured person about stimulation from the electrode pair within a specific range of the switching period and/or plural points of stimulation felt by the measured person about stimulation from the electrode pair within other specific range of the switching period. Namely, it is possible to be inspect a neural resolution (called as double- points discrimination proficiency) while the measured person wears the electrode sheet only one time without doing it again. Accordingly, time of measuring a neural resolution can be shortened. Further, a method of measuring a neural resolution can be simplified.

In order to overcome the above issue, according to yet other aspect of the invention, it is a method of controlling a stimulation-current providing device that includes a plurality of electrode pairs, each having a pair of positive and negative electrodes and arranged on a electrode sheet in order, and provides stimulation current for a measured person by applying a voltage to the plurality of electrode pairs. The method comprises: measuring a minimum- perception current value by applying a pulse -like voltage to any of the electrode pairs, the pulse -like voltage having a predetermined initial switching period in order to gradually increase the stimulation current, and the minimum- perception current value being a current value of the stimulation current at the time when the measured person feels initial stimulation so as to gradually increase the stimulation current (a step of measuring a threshold value); and applying the pulse-like voltage to the electrode pair while gradually extending or shortening the switching period, compared to an initial switching period and shifting timing (on time) of raising the voltage applied to the plurality of electrode pairs each other (a voltage application step).

The method of the stimulation-current providing device in the yet other aspect of the invention includes: applying a pulse- like voltage to the electrode pair in the voltage application step, while gradually extending or shortening the switching period, compared to an initial switching period and shifting timing (on time) of raising the voltage applied the plurality of pair electrode each other. Hence, it is possible to measure one point of stimulation felt by the measured person about stimulation from the pair electrode within a specific range of the switching period and plural points of stimulation felt by the measured person about stimulation from the electrode pair within other specific range of the switching period. Namely, it is possible to inspect a neural resolution while the measured person wears the electrode sheet only one time without doing it again. Accordingly, time of measuring a neural resolution can be shortened. Further, a method of measuring a neural resolution can be simplified.

In order to overcome the above issue, according to yet other aspect of the invention, a stimulation-current providing device comprises: an electrode sheet that includes a plurality of electrode pairs and is installed on a measured person, each of the electrode pairs being composed of a pair of negative and positive electrodes: a voltage application unit that applies a voltage the electrode pairs so as to apply a stimulation current to the measured person from the electrode pairs. The plurality of electrode pairs are arranged on the electric sheet so as to be aligned in order from a central nerve to a peripheral nerve of the measured person. The voltage application unit applies a voltage to every one of the plurality of electrode pairs in the order from one end of the electrode -pairs to the other end of it so as to provide a stimulation current for the measured person.

The stimulation-current providing device of yet other aspect of the invention includes plurality of electrode pairs that are arranged on the electric sheet so as to be aligned in order from a central nerve to a peripheral nerve of the measured person. Further, in the device, the voltage application unit applies a voltage to every one of the plurality of electrode pairs in the order from one end of the electrode -pairs to the other end of it so as to provide a stimulation current for the measured person. Namely, it is possible to inspect a peripheral -nerve damage while the measured person wears the electrode sheet only one time without doing it again. Accordingly, time of measuring a peripheral - nerve damage can be shortened. Further, measuring a peripheral-nerve damage can be simplified.

In order to overcome the above issue, according to yet other aspect of the invention, a method of controlling a stimulation-current providing device comprises:
providing a stimulation current for a measured person from a plurality of electrode pairs by applying a voltage to every one of plurality of electrode pairs in the order from one end of electrode -pairs arrangement to the other end of them (a step of providing a current). Each of the electrode pairs includes positive and negative electrode, and are arranged on an electric sheet so as to be aligned in order from a central nerve to a peripheral nerve of the measured person.

The method of controlling a stimulation-current providing device of yet other aspect of the invention provides the stimulation current for the measured person from the plurality of electrode pairs in the step of providing the current by applying the voltage to every one of the plurality of electrode pairs in the order from one end of electrode -pairs to the other end of them, the electrode pairs including a pair of positive and negative electrodes, and being arranged on an electric sheet so as to be aligned in order from a central nerve to a peripheral nerve of the measured person. Namely, it is possible to be measure a peripheral -nerve damage while the measured person wears the electrode sheet only one time without doing it again. Accordingly, time of measuring a peripheral - nerve damage can be shortened and the method of it can be simplified.

In order to overcome the above issue, according to yet other aspect of the invention, it is a method of controlling a stimulation-current providing device that includes at least three or more pluralities of electrode pairs, each composed of a pair of positive and negative electrodes and arranged on a electrode sheet in order, and applies a voltage to so as to provides a stimulation current for a measured person. The method comprises: defining a specific electrode pair among a plurality of electrode pairs arranged on a electrode sheet as the standard electrode pair, the specific electrode being placed at the location far from the end of the arranged plurality of electrode pairs by at least two electrode pairs; measuring a minimum- perception current value that is a stimulation current value at the time when the measured person feels initial stimulation while applying a voltage to a standard electrode pair so as to gradually increase the stimulation current (a step for measuring a perception- threshold value for the standard electrode pair); applying a voltage to the standard electrode pair so as to provide a stimulation current of the minimum- perception current value after the step for measuring a perception- threshold value for the standard electrode pair, while applying a voltage to any of the plurality of electrode pairs in series and a way of approaching to the standard electrode pair or stepping away from the standard electrode pair so as to provide a stimulation current of the minimum- perception current value (a voltage application step). The plurality of electrode pairs are arranged in a manner that any of the electrode pairs be located at a position far from the standard electrode pair by at least two or more electrode pairs and others of the electrode pairs be arranged in series and way of approaching the standard electrode pair.

In the method of controlling the stimulation-current providing device, a voltage is applied to the standard electrode pair in a step of voltage application. Further, the method applies a voltage to any of the plurality of electrode pairs in series and in a way of approaching to the standard electrode pair or stepping away from the standard electrode pair in order so as to provide a stimulation current of the minimum- perception current value (a voltage application step). The plurality of electrode pairs are arranged in a manner that any of the electrode pairs is located at a position far from the standard electrode pair by at least two or more electrode pairs and others of the electrode pairs are arranged in series and way of approaching the standard electrode pair. Namely, it is possible to inspect a neural resolution while the measured person wears the electrode sheet only one time without doing it again. Accordingly, time of measuring a neural resolution can be shortened. Further, a method of measuring a neural resolution can be simplified.

Further, the method applies a voltage to the standard electrode pair in the step of voltage application so as to provide the stimulation current of the minimum- perception current value measured at the step of measuring a step for measuring a perception-threshold value for the standard electrode pair. Hence, it is possible to objectively inspect a neural resolution by using the minimum- perception current value as a standard. Further, nerves of the measured people are not excessively stimulated since the stimulation current of the minimum- perception current value flows in the people. Accordingly, it is possible to accurately inspect a neural resolution thereby.

In the method of the above yet other aspect of the invention, the phases of these two voltages applied to electrode pairs may be preferably shifted by approximately 180°. These two voltages are pulse- like voltages during the step of voltage application; the one is applied to the standard electrode pairs and the other is applied to one of the plurality of electrode pairs in the order of approaching to the standard electrode pair, the plurality of electrode pairs being arranged in the order from the electrode pair located at a position far from the standard electrode pair by at least two or more electrode pairs toward the standard electrode pair.

The above arrangement can avoid the interference of the stimulation current provided for the measured people from the standard electrode pair with the stimulation current provided for the measured people from the a plurality of electrode pairs arranged in order toward the standard electrode pair. Hence, it is possible to appropriately measure a neural resolution.

In order to overcome the above issue, according to yet other aspect of the invention, a stimulation-current providing device comprises: an electrode sheet that includes a plurality of at least three or more electrode pairs and is installed on a measured person, each of the electrode pairs being composed of a pair of negative and positive electrodes: a voltage application unit that applies a voltage to the electrode pairs so as to apply a stimulation current to the measured person from the electrode pair.

The plurality of electrode pairs are arranged in order on the electrode sheet. When a specific electrode pair among a plurality of electrode pairs arranged on a electrode sheet is defined as the standard electrode pair, the specific electrode being placed at the location far from the end of the arranged plurality of electrode pairs by at least two electrode pairs, first, the voltage application unit applies a voltage to the standard electrode pair. Then, the voltage application unit applies a stimulation current to the measured person by applying a voltage to the standard electrode pair while applying a voltage to any of the plurality of electrode pairs in series and a way of approaching to the standard electrode pair or stepping away from the standard electrode pair. The plurality of electrode pairs are arranged in a manner that any of the electrode pairs be located at a position far from the standard electrode pair by at least two or more electrode pairs and others of the electrode pairs be arranged in series and way of approaching the standard electrode pair.

The stimulation-current providing device according to the yet other aspect of the invention applies a voltage to the standard electrode pair so as to apply a stimulation current to the measured person. Further, the device applies a voltage to any of the plurality of electrode pairs in series and a way of approaching to the standard electrode pair or stepping away from the standard electrode pair. The plurality of electrode pairs are arranged in a manner that any of the electrode pairs be located at a position far from the standard electrode pair by at least two or more electrode pairs and others of the electrode pairs be arranged in series and way of approaching the standard electrode pair. As a result, it is possible to inspect a neural resolution while the measured person wears the electrode sheet only one time without doing it again. Accordingly, time of measuring neural resolution can be shortened and, a method of it can be simplified.

Further, in the yet aspect of the invention, first, the device applies a voltage to the standard electrode pair before inspecting a neural resolution. Hence, it is possible to measure the minimum- perception current value provided by the standard electrode pair. Namely, it is possible to provide the stimulation current of the minimum-perception current value based on the measurement result when inspecting a neural resolution. As a result, it is possible to objectively inspect a neural resolution by using the minimum- perception current value as a standard. Further, accurate measurement of a neural resolution can be available since nerves of the measured person are not excessively stimulated when the stimulation current of the minimum- perception current value is provided for the person.

In order to overcome the above issue, according to yet other aspect of the invention, a stimulation-current providing device comprises: an electrode sheet that includes a plurality of electrode pairs and is installed on a measured person, each of the pair electrodes being composed of a pair of negative and positive electrodes: and a voltage application unit that applies a voltage to the electrode pairs so as to apply a stimulate current to the measured person from the electrode pairs. The at least two electrode pairs are arranged on the electrode sheet so that the pair of the negative and positive electrodes are aligned without overlapping two or more regions of a skin perceptual band of the measured person.

In the yet other aspect of the invention, the at least two electrode pairs are arranged on the electrode sheet so that the pair of the negative and positive electrodes are aligned without overlapping two or more regions of a skin perceptual band of the measured person. Namely, it is possible to be measure neural situations in plural regions of a skin perceptual band while the measured person wears the electrode sheet only one time without doing it again. Accordingly, time of measuring neural situations in plural regions of a skin perceptual band can be shortened and, a method of it can be simplified.

In order to overcome the above issue, according to yet other aspect of the invention,: it is a method of controlling a stimulation-current providing device that includes a plurality of electrode pairs, composed of a pairs of positive and negative electrodes and arranged on a electrode sheet in order, and applies a voltage to the plurality of electrode pairs so as to provides a stimulation current for a measured person. The method comprises a step of providing a stimulation current for the measured person by applying a voltage to the at least two electrode pairs in order. These electrodes include the pair of the negative and positive electrodes that are aligned without overlapping two or more regions of a skin perceptual band of the measured person.

The method of the yet other aspect of the invention provides a stimulation current for the measured person by applying a voltage to the at least two electrode pairs in order. These electrodes include the pair of the negative and positive electrodes that are aligned without overlapping two or more regions of a skin perceptual band of the measured person. Namely, it is possible to inspect neural situations in plural regions of a skin perceptual band while the measured person wears the electrode sheet only one time without doing it again. Accordingly, time of measuring neural situations in plural regions of a skin perceptual band can be shortened and, a method of it can be simplified.

### Advantage of the Invention

According to the above mentioned aspects of the invention regarding the stimulation-current providing device and a method of controlling the same, it is possible to appropriately inspect a neural damage. Further, these aspects of the invention regarding the stimulation-current providing device and a method of controlling the same, it is possible to shorten inspection time.

### Brief Description of Drawings

- Fig. 1: is a perspective view of a stimulation-current providing device according to a first embodiment of the invention.
- Fig. 2: is a block chart showing a schematic view of a body of the stimulation-current providing device shown in Fig. 1 and peripheral devices thereof.
- Fig. 3: is a diagram showing voltage waveforms outputting from an output control circuit shown in Fig. 2.
- Fig. 4: is a flow chart showing inspection procedures regarding to a neural damage using the stimulation-current providing device shown in Fig. 1.
- Fig. 5: is a diagram showing a state of a measured person who wears an electrode sheet regarding second embodiment of the invention
- Fig. 6: is a flow chart showing a part of procedures inspecting a peripheral -nerve damage of with using the electrode sheet shown Fig. 5.
- Fig.7: is a schematic view explaining the inspection of two-points discrimination proficiency.
- Figs. 8(A) and 8(B): are diagrams showing a voltage applied to the electrode pair shown in Fig. 5 during the inspection of two-points discrimination proficiency.
- Fig. 9: is a flow chart showing a part of procedures inspecting two-points discrimination proficiency with using the electrode sheet shown Fig. 5.
- Fig. 10: is a diagram showing a skin perceptual band.
- Fig. 11: is a diagram showing a state of a measured person who wears the other electrode sheet regarding the second embodiment of the invention.
- Fig. 12: is a flow chart showing a part of procedures measuring a minimum- perception current value in a plurality of regions of the skin perceptual band with using the electrode sheet shown Fig. 11.
- Fig. 13: is a diagram showing a state of a measured person who wears the other electrode sheet regarding the second embodiment of the invention.
- Fig. 14: is a diagram showing voltage waveforms applied to a modification 1 of the second embodiment of the invention.

### Reference Numerals

### 1: stimulation current proving device,

2: person to be measured
3:E,E(n) to E(n+3), E(1) to E(4), E10, E11, electrode pair
3a; negative electrode
3b: positive electrode
10: boosting transformer (a part of voltage application unit)
11. voltage control circuit (a part of voltage application unit)
12. output control circuit (a part of voltage application unit)
31. negative electrode
32: positive electrode,
33,43 and 53 ;electrode sheet
R1 to R7: region,
S2: step of measuring threshold value
S3: step of applying voltage,
S22: a part of applying current and a part of measuring threshold value,
S23: a part of measuring threshold value
S24: a part of applying current and a part of measuring threshold value,
S25: a part of measuring threshold value
S26: a part of applying current and a part of measuring threshold value,
S32, S33 measuring a perception- threshold value for the standard

### electrode pair,

S34 to S36: step of applying voltage,
S42 and S44: step of applying current,
T0: switching period,
t0: on time

### The preferred embodiments of the invention

Embodiments of the invention will be described with reference to the accompanying drawings.

### Embodiment 1

### Schematic View of Stimulation-Current Providing Device

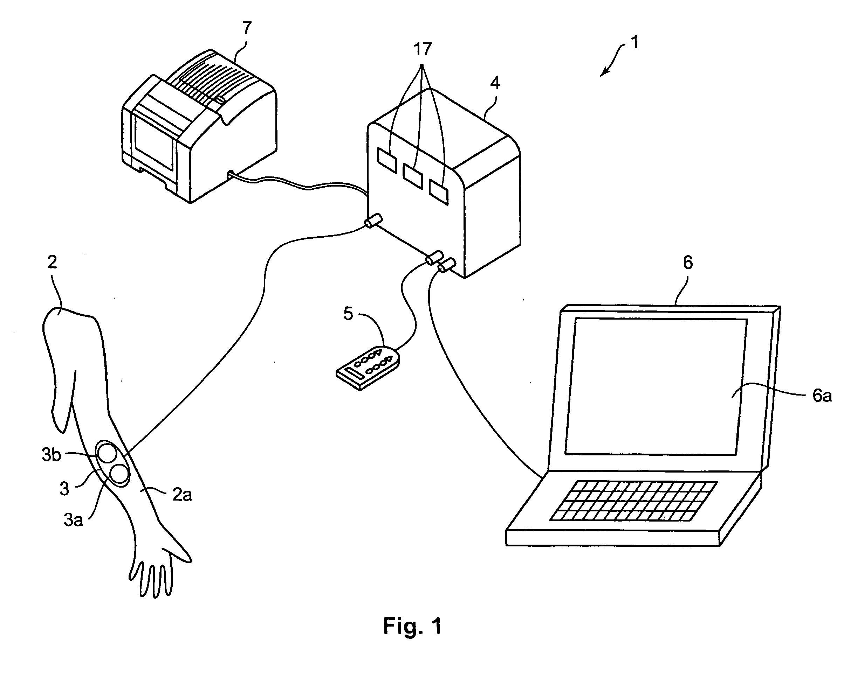

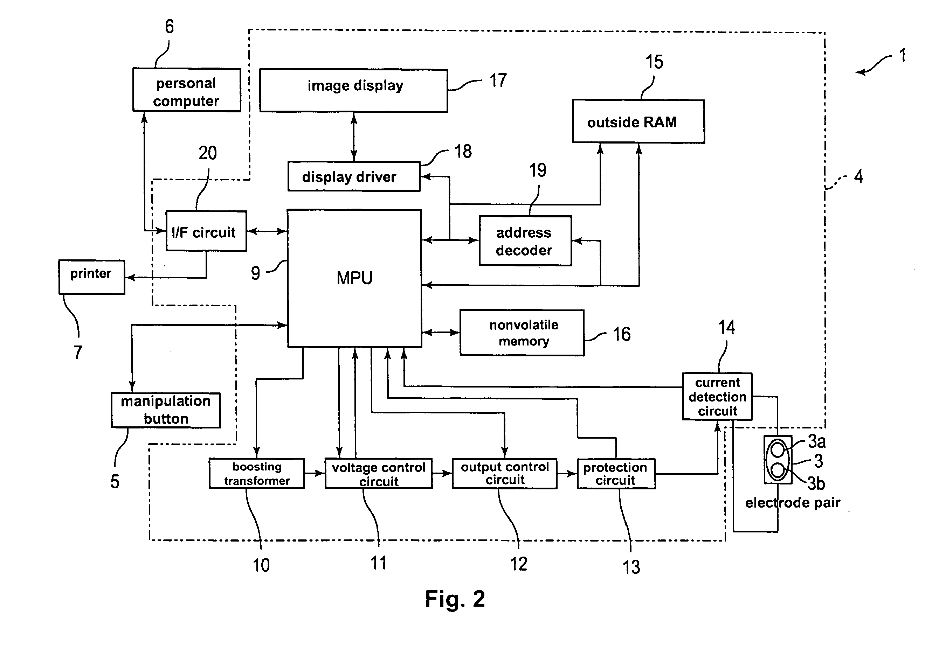

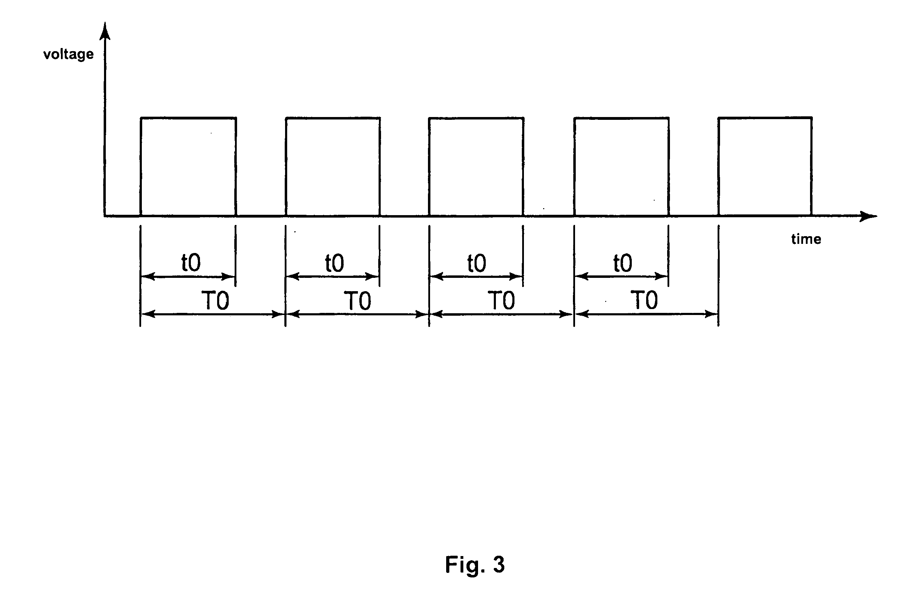

Fig. 1 is a perspective view of a stimulation-current providing device 1 according to a first embodiment of the invention. Fig. 2 is a block chart showing a schematic view of a body 4 of the stimulation-current providing device 1 shown in Fig. 1 and peripheral devices thereof. Fig. 3 is a diagram showing voltage waveforms outputting from an output control circuit 12 shown in Fig. 2.

The stimulation-current providing device 1of the embodiment is to provide a stimulation current for a measured person 2 and inspect a neutral damage for the measured person 2. As shown in Fig. 1, the stimulation-current providing device 1comrpsies an electrode pair 3, the body 4, an operational button 5, a personal computer (PC) 6 and a printer 7. The electrode pair 3 is installed inside of an upper arm portion 2a of the measured person 2. The operational button 5 is operated by the measured person 2 at the time of inspection. PC 6 applies predetermined signals to the body 4 and displays a inspection result. The printer 7 prints the inspection result on a printing paper and outputs it. The electrode pair 3, the operational button 5, PC6 and the printer 7 are connected to the body 4 by a given cable. The electrode pair 3 is composed of a pair of a negative electrode 3a and a positive electrode 3b.

In Fig. 1, the electrode pair 3 is installed inside of an upper arm portion 2a of the measured person 2. Otherwise, the electrode pair 3 may be installed in or on other places in which amounts of muscles and sweat glands are small and the electrode pair is easily wearable.

As shown in Fig. 2, the body 4 comprises a micro processing unit (MPU) 9, a boosting transformer 10, a voltage control circuit 11, an output control circuit 12 , a current detection circuit 14, an outside RAM 15, a nonvolatile memory 16, an image displaying unit 17, a displaying driver 18, and address decoder 19 and an interface (IF) circuit 20.

MPU 9 includes ROM, RAM, a timer, output interfaces and the like inside. ROM inside of MPU9 stores programs for operating predetermined processes. When MPU 9 receives an operational signal from PC6 via the I/F circuit 20, MPU9 processes the operational signal from PC 6 following a program stored in ROM with using a temporary memory in the outside RAM 15 and executes a predetermined algorism. Further, MPU 9 provides a driving signal for the boosting transformer 10, the voltage control circuit 11 and the output control circuit 12 by executing the predetermined algorism.

The boosting transformer 10 boosts a voltage from a DC current source not shown in the figure depending on the driving signal from MPU9. More specifically, the boosting transformer 10 drives transistors with a rectangular- shaped drive signal from MPU9 using a timer and boosts a voltage from the DC current source. For example, the he boosting transformer 10 boosts 12 V voltage given by the DC current source to 100-120V.

The voltage control circuit 11 regulates an output voltage output from the boosting transformer 10 depending on the drive signal from MPU9. Further, as shown in Fig. 2, the voltage control circuit 11 outputs a detection signal for detecting a voltage value at the voltage control circuit 11 to MPU9. MPU9 controls the voltage control circuit 11 not to output a voltage exceeding a predetermined value based on the detecting signal input to MPU9.

The output control circuit 12 is a PWM control circuit controlling a rectified voltage output from the voltage control circuit 11 via pulse width modulation (PWM.) More specifically, the output control circuit 12 converts the rectified voltage output from the voltage control circuit 11 into a pulse -like voltage having a predetermined switching period T0 shown in Fig. 3 depending on the driving signal from MPU9 and output it. For example, the output control circuit 12 outputs the pulse -like voltage in the range of 5V to 100V. As shown in Fig. 3, the output control circuit 12 outputs a predetermined voltage during the predetermined on time "t0" in the embodiment.

The protection circuit 13 is a limiter circuit to prevent the electrode pair 3 from providing a current exceeding a predetermined value for the measured person 2. Further, as shown in Fig. 2, the voltage control circuit 13 outputs a detection signal for detecting a current limited value to MPU9.

The current detecting circuit 14 is to detect (measure) an effective value of the stimulation current provided for the measured person 2 via the electrode pair 3. The current detecting circuit 14 of the embodiment comprises resistors, an operational amplifier and the like for example. As shown in Fig. 2, the current detecting circuit 14 outputs a detecting signal to MPU9. The detecting signal detects the current value of the stimulation current provided for the measured person from the electrode pair 3.

Here, in the embodiment, a voltage application unit comprises the boosting transformer 10, the voltage control circuit 11 and the output control circuit 12 and applies a voltage to the electrode pair 3 to provide the stimulation current for the measured person 2 from the electrode pair 3. The voltage application unit outputs the pulse -like voltage described above. Namely, the voltage application unit applies the voltage to the electrode pair 3 by a PWM method that applies a pulse -like voltage having the predetermined switching period T0.

The outside RAM 15 is a memory for algorism to be executed by MPU9. The non volatile memory 16 is a memory storing a predetermined -set value such as a limit value of the voltage applied to the current detection circuit 14.

The image displaying unit 17 is a display such as a liquid crystal display showing the value of stimulation current given to the measured person 2 from the electrode pair 3 and the switching period T0 of the voltage given to the electrode pair 3 (namely frequency of the voltage) shown in Fig. 1. The image displaying unit 17displays image data output from MPU 9 and processed by the display driver 18.

An address decoder 19 is a logic circuit that exchanges signals among the outside RAM 15 , display driver 18 and MPU9. The I/F circuit 20 is a circuit that exchanges signals between MPU9 and PC6, and supplies signals from MPU9 to the printer 7.

The manipulation button 5 includes a stop switch to stop application of the voltage to the electrode pair 3 by the measured person 2 and a start switch to start the application of the voltage. The manipulation button 5 further includes a selection button for the measured person to select whether he or she feels continuous stimulation or oscillation (intermittent) stimulation about the stimulation current given to him or her by the electrode pair 3 at the time of inspecting a neural damage.

PC6 includes a display 6a displaying inspection results from the stimulation-current providing device 1 as shown in Fig. 1. The display is a liquid crystal display for example.

### Method of Inspecting Neural Damage

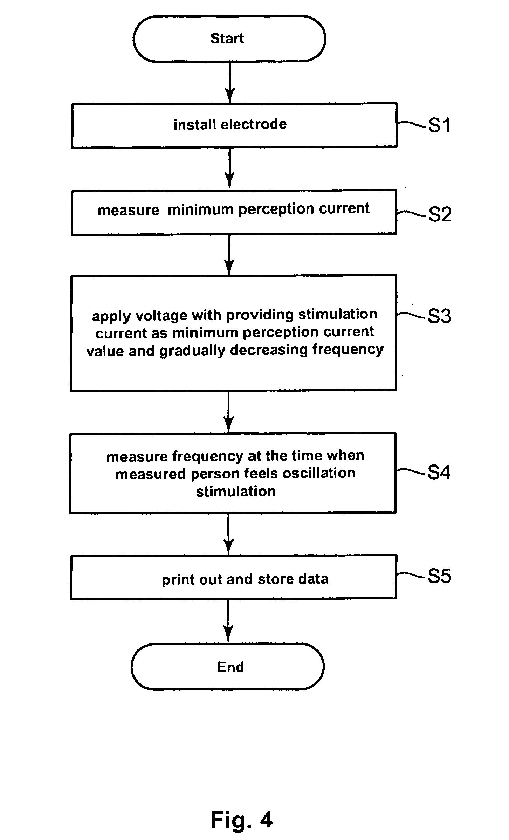

Fig. 4 is a flow chart showing inspection procedures regarding to a neural damage using the stimulation-current providing device 1 shown in Fig. 1.

A method of inspecting a neural damage using the stimulation-current providing device 1 will be explained.

A neural damage regarding the measured person 2 is inspected by the following; when changing the switching period T0 of the voltage applied to the electrode pair 3 (changing voltage frequency), the interface between the switching period T0 (frequency) at the time of continuous stimulation felt by the measured person 2 and the switching period T0 (frequency) at the time of oscillation (intermittent) stimulation is measured. Then, a measured value corresponding to neural transmission speed is obtained based on the measured frequency interface.

For example, the neural transmission speed decreases, as being judged as some progress of a neural damage in a case when the measured person does not feel the oscillation stimulation based on the stimulation current if the frequency of the voltage applied to the electrode pair 3 does not decrease. On the other hand, the neural transmission speed is normal, being judged as no neural damage in a case when the measured person feels the oscillation stimulation based on the stimulation current if the frequency of the voltage applied to the electrode pair 3 is relatively high.

The flow chart shown in Fig. 4 shows the procedure of inspecting a neural damage using the stimulation-current providing device 1 for example. First, the measured person 2 wears the electrode pair 3 (step S1.) Then, a voltage is applied to the electrode pair 3 so as to gradually increase the current value of the stimulation current given to the measured person 2 from "0" (so as to gradually increase the voltage applied to the electrode pair 3 from "0"). Next, the measured person 2 pushes the stop switch of the manipulating button 5 held with his or her hand if he or she initially feels the stimulation current. Pushing the stop switch halts application of a voltage to the electrode pair 3 and makes MPU9 memorize the current value at that time (step S2.)

Here, the current value of the stimulation current measured by step S2 is a standard current value for inspection (a perception- current threshold value.) This current value is defined as "a minimum- perception current value".

Next, a voltage is applied to the electrode pair 3 so that the stimulation current of the minimum-perception current value is applied to the measured person 2 from the electrode pair 3 and the frequency of an applied voltage is gradually decreased (the switching period T0 of the voltage is gradually increased) (step S3). Namely, step S3 applies a voltage to the electrode pair 3 by gradually increasing the switching period T0 compared to the initial switching period T0 of the voltage at the time of measuring the minimum-perception current value (gradually decreasing frequency compared to the initial frequency of a voltage at the time of measuring the minimum-perception current value). Here, the initial frequency is a frequency in which the measured person 2 feels continuous stimulation based on the stimulation current given to the electrode pair 3 and 50Hz for example.

When step S3 applies a voltage to the electrode pair 3 by gradually decreasing the frequency and the measured person 2 who has felt the continuous stimulation based on the stimulation current feels the oscillation current based on the stimulation current, the measured person 2 pushes the selection button of the manipulation button held with his or her hand. Pushing the selection switch halts application of a voltage to the electrode pair 3 and makes MPU9 memorize the frequency at that time (step S4). Then, the measured result of the frequency is printed on a paper by the printer and stored in PC6 (step S5). Further, the inspection value corresponding to neural transmission speed is obtained by the stored data. The inspection regarding a neural damage is completed.

In the embodiment, step S3 is a step of measuring the threshold value, the minimum-perception current value, by applying a pulse -like voltage having the predetermined switching period T0 to the electrode pair 3 so as to gradually increase the stimulation current. Further, step 3 is a step of applying a pulse -like voltage to the electrode pair 3 while gradually increasing the switching period T0 of the applied voltage compared to the initial switching period T0.

### Major Advantages of Embodiment 1

As described above, in the embodiment, step S3 applies a pulse -like voltage to the electrode pair 3 while gradually increasing the switching period T0 of the applied voltage compared to the initial switching period T0. Hence, in the aspect of the invention, it is possible to inspect continuous stimulation felt by the measured person 2 about the stimulation from a pair electrode within a specific range of the switching period T0 and oscillating (intermittent) stimulation felt by the measured person 2 about the stimulation from a pair electrode 3 within other specific range of the switching period T0. Namely, the invention is able to obtain measured values corresponding to neural transfer speeds. Hence, the invention is able to appropriately inspect a neural damage.

### Modification of Embodiment 1

In the embodiment described above, step S3 applies a voltage to the electrode pair while gradually increasing the switching period T0. Instead, step S3 may apply a voltage to the electrode pair 3 while gradually decreasing the switching period T0 (namely gradually increasing frequency of the voltage). In this case, the initial frequency of the voltage at the time of measuring the minimum-perception current value is a frequency where the measure person 2 feels the oscillation stimulation based on the stimulation current received from the electrode pair 3.

### Embodiment 2

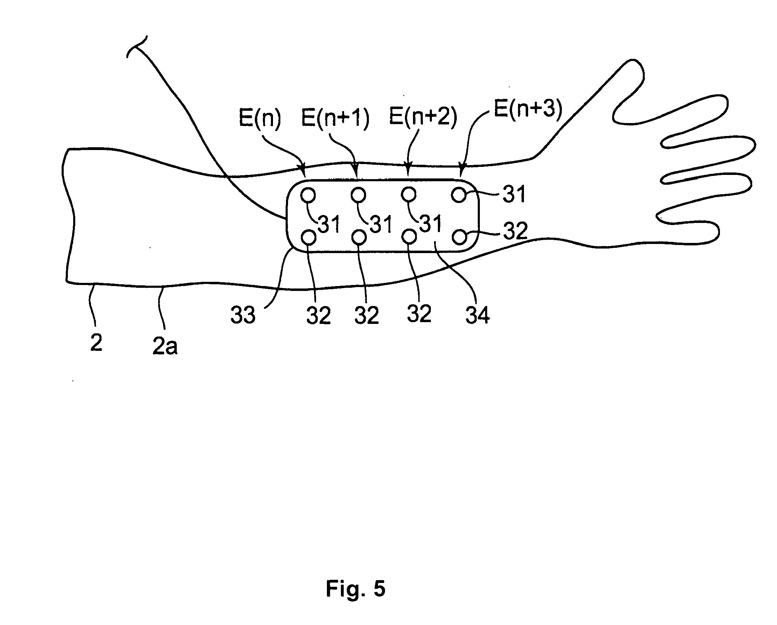

The major difference between the embodiments 1 and 2 is as following; the measured person 2 wears the electrode pair 3 comprising a pair of the positive and negative electrodes in the embodiment 1. On the other hand, in the embodiment 2, the measured person 2 wears the electrode sheets 33, 43, and 53 shown in Figs. 5, 11 and 13, comprising a plurality of electrode pairs E, each including a pair of negative and positive electrodes 31 and 32. Further, as other difference, various neural damages are inspected in the embodiment 2. Hence, electrode sheets 33 and others and various inspections will be explained hereafter. Further, a method of controlling the stimulation-current providing device 1 will be explained at the time of various inspections.

### Electrode Sheet Structure Used for Inspecting Damage of Peripheral Nerve and Procedure for Inspecting Damage of Peripheral Nerve

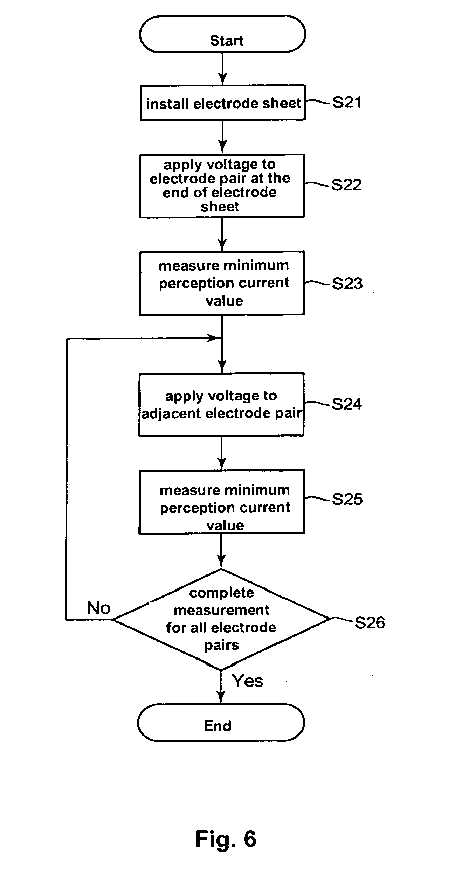

Fig. 5 is a diagram showing a state of the measured person 2 who wears the electrode sheet 33 regarding second embodiment of the invention Fig. 6 is a flow chart showing a part of procedures inspecting a damage of peripheral nerves with using the electrode sheet 33 shown in Fig. 5.

It is possible to inspect a damage of peripheral nerves for a diabetic by using the stimulation-current providing device 1 of the embodiment. When the stimulation-current providing device 1 is used for inspecting a damage of peripheral nerves, the measured person 2 wears the electrode sheet 33 shown in Fig. 5.

The electrode sheet 33 comprises a plurality of electrode pairs E including a pair of the negative and positive electrodes 31 and 32 and a base sheet 34 fixing the plurality of electrode pairs E. In Fig. 5, the electrode sheet 33 is provided with four electrode pairs E(n) to E(N+3). The base sheet 34 has an approximately rectangular shape. The numbers of electrode pairs are not limited to four, may be any numbers equal to or more than two.

The electrode pairs E(n) to E(n+3) are adjacently arranged each other in order with a predetermined interval from the one end to the other end of the electrode sheet 33. In the embodiment, as shown in Fig. 5, the electrode pairs E(n) to E(n+3) are arranged toward the horizontal direction of the base sheet 34 so that the electrode pairs E(n) to E(n+3) are able to be aligned from a central nerve ( a spinal cord) of the measured person 2 to a peripheral nerve. The interval between two electrode pairs of E(n) to E(n+3) is 10 mm to 20mm for example.

The electrode sheet 33 is connected to the body 4 shown in Fig. 1 and the like. More specifically, each of the electrode pairs E is connected to the voltage application unit comprising the boosting transformer 10, the voltage control circuit 11 and the output control circuit 12 via the protection circuit 13 and the current detection circuit 14. In the embodiment, it is possible for the voltage application unit to apply a voltage to arbitrary one of the electrode pairs E so as to give the stimulation current to the measured person 2.

Here, in the example of Fig. 5, the measured person 2 wears the electrode sheet 33 with the upper arm 2a. But, the measured person 2 may wear the electrode sheet 33 with his or her leg so that the electrode pairs E(n) to E(n+3) are able to be aligned from a central nerve of the measured person 2 to a peripheral nerve.

When inspecting a damage of spherical nerves using the electrode sheet 33, voltages are applied to the electrode pairs E(n) to E(n+3) in series as the order of E(n) to E(n+3) or the vice versa. Further, each of the minimum- perception current values is measured in order at the time when each of the electrode pairs E(n) to E(n+3) gives the stimulation currents to the measured person 2. Then, the minimum- perception currents are compared with the standard current value measured and set in advance, specifying any of electrode pairs E that provided the stimulation current which is equal to or more than the standard current value. Specifying any of electrode pairs E, provided the stimulation current which is equal to or more than the standard current value, recognizes the progress of a neural damage from the center to peripherals.

Fig. 6 shows a procedure of measuring the minimum-perception currents for inspecting a damage of peripheral nerves using the electrode sheet 33 as an example. First, the measured person 22 wears the electrode sheet 33 so that the electrode pairs E(n) to E(n+3) are aligned from a central to peripheral nerves (step S21). Second, a voltage is applied to the electrode pair E(n) located at the end of the electrode sheet 33 so that the current value of the stimulation current given to the measured person 2 is gradually increased from "0" (step S22). Next, the measured person 2 pushes the stop switch of the manipulating button 5 held with his or her hand if he or she initially feels the stimulation current. Pushing the stop switch makes MPU 9 memorize the current value at that time as the minimum- perception current.

Next, a voltage is applied to the electrode pair E(n+1) located adjacent to the electrode pair E(n) so that the current value of the stimulation current given to the measured person 2 is gradually increased from "0" (step S24.) Next, the measured person 2 pushes the stop switch of the manipulating button 5 held with his or her hand if he or she initially feels the stimulation current. Pushing the stop switch makes MPU 9 memorize the current value at that time as the minimum- perception current (step S25).

Then, completing the measurement of all minimum- perception currents of electrode pairs E(n) to E(n+3) is judged (step S26). The procedure gets back to step S24 if the measurement of all minimum- perception currents of electrode pairs E(n) to E(n+3) is not completed. Accordingly, the measurement of all minimum- perception currents of electrode pairs E (n) to E (n+3) is completed. Further, step S26 finishes the measurement of the minimum- perception current if the step judges completing the measurement of all minimum- perception currents of electrode pairs E (n) to E (n+3).

Here, all procedures from step S22 to S26 are controlled by MPU9 and the like. But, the measured person 2 or a medical doctor may operate the stimulation-current providing device 1 so as to apply voltages to electrode pairs E(n) to E(n+3) in series. Otherwise, the minimum- perception currents may be measured with three electrode pairs E(n) to E(n+2) at the time when the stimulation currents are given by these three electrode pairs instead of all four electrode pairs E(n) to E(n+3).

In the embodiment, steps S22, S24 and S26 are to apply voltages to electrode pairs E(n) to E(n+3) in series as an order from one end to the other of a plurality of electrode pairs E(n) to E(n+3) so as to provide the stimulation currents for the measured person from the electrode pairs E(n) to E(n+3).

In the embodiment described above, the plurality of electrode pairs E are arranged in the electrode sheet 33 so that the electrode pairs are able to be aligned from the central to peripheral nerves of the measured person 2. Then , voltages are applied to these electrode pairs in the order from the arranged one end to the other end so as to provide the stimulation currents to the measured person 2. Accordingly, it is possible to measure the minimum- perception currents from a central to peripheral nerves while the measured person wears the electrode sheet 33 one time without doing it again. Further, it is possible to inspect a damage of peripheral nerves of the measured person 2 based on the measured minimum- perception current values. Accordingly, time of measuring damage of peripheral nerves can be shortened and, a method of it can be simplified.

### Electrode Sheet Structure Used for Two-points Discrimination Proficiency and Procedure for Inspecting Damage of Peripheral Nerve

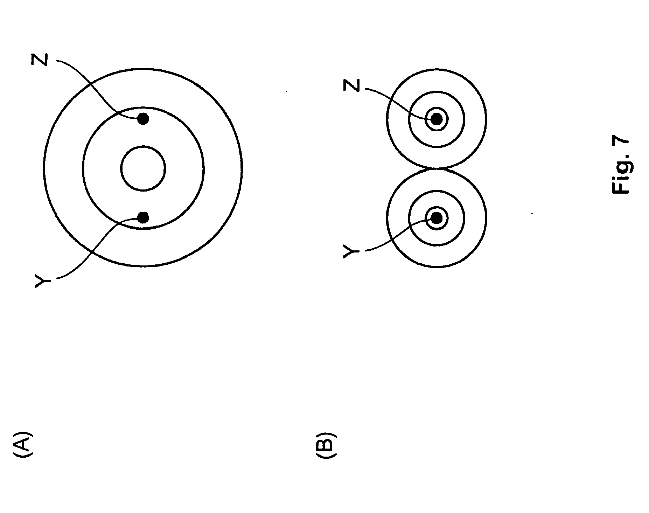

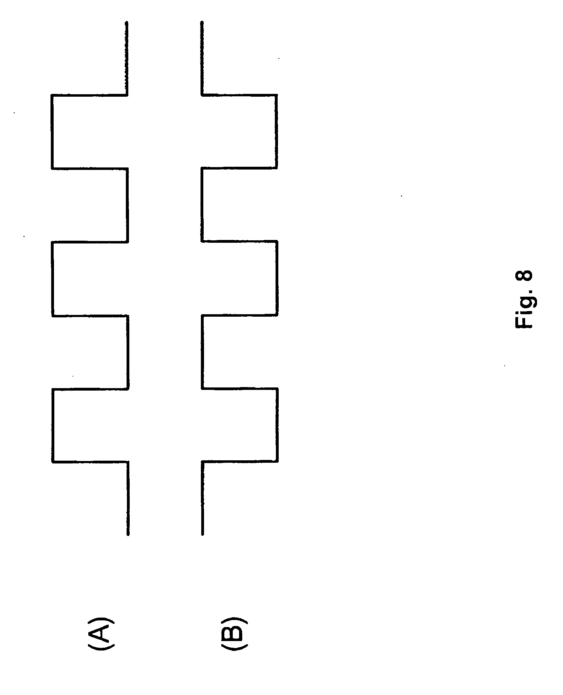

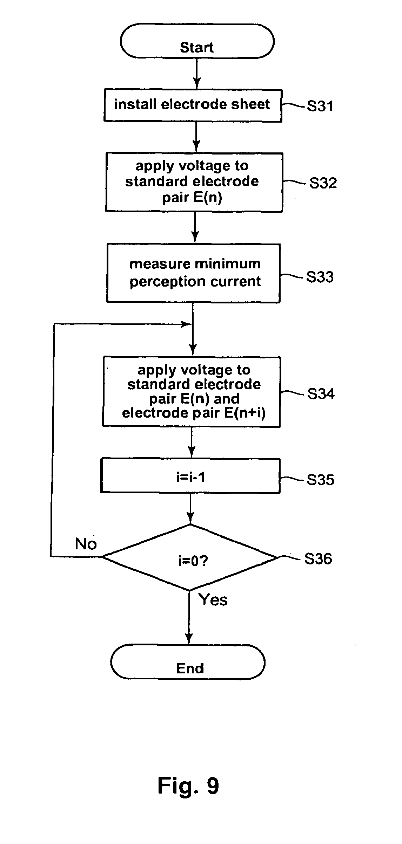

Fig. 7 is a schematic view explaining the inspection of two-points discrimination proficiency. Figs. 8(A) and 8(B) are diagrams showing voltages applied to the electrode pairs E shown in Fig. 5 during the inspection of two-points discrimination proficiency. Fig. 9 is a flow chart showing a part of procedures inspecting two- points discrimination proficiency with using the electrode sheet 33 shown Fig. 5.

It is possible to inspect two- points discrimination proficiency (tactile sensibility identifying two points on a surface of a body simultaneously touched with anything else) by using the stimulation-current providing device 1 of the embodiment. Namely, as shown in Figs. 7(A) and (B), when stimulation is given to two points Y and Z on a body surface of the measured person 2 at the same time, it is possible to inspect whether the measured person feels one -point stimulation shown as Fig. 7(A) or two- points stimulation shown as Fig. 7(B) by using the stimulation-current providing device 1 of the embodiment. Here, inspecting inspect two- points discrimination proficiency make it possible to diagnose a brain cortex (more specifically, a parietal lobe damage) for example.

When the stimulation-current providing device 1 is used for inspecting two-points discrimination proficiency, the measured person 2 wears the electrode sheet 33 shown in Fig. 5. Further, each of a plurality of electrode pairs E is connected to the voltage application unit. Applying a voltage to any of electrode pairs E make it possible for each of electrode pairs E to apply the stimulation current to the measured person 2. Here, it is necessary that the electrode sheet 33 used for inspecting two-points discrimination proficiency comprises at least three or more electrode pairs E.

In case of inspecting two-points discrimination proficiency using the electrode sheet 33, first, a voltages are applied to two electrode pairs E such as E(n) and E(n+3) while the electrode pair E(n) being as a standard electrode pair. Next, voltages are applied to two electrode pairs E such as E (n) and E(n+2). Then, voltages are applied to two electrode pairs E such as E (n) and E (n+1). Namely, voltages are applied to the electrode pairs E(n+3) to E(n+1) in series and in a way of approaching to the electrode pair E(n) while a voltage is also applied to the electrode pair E(n) as a standard. The electrode pairs are arranged in a manner that the electrode pair E(n+3) is located at a position of the most far from the electrode pair E(n) as a standard and other electrode pairs are aligned toward the electrode pair E(n).

Further, when the measured person 2 receives the stimulation currents by applying voltages to two electrode pairs E among the above mentioned combination, the device inspects whether the measured person 2 feels one- point stimulation or two- points stimulation. For example, a stimulation selecting switch is installed in the manipulation button 5. Pushing the switch by the measured person 2 records whether the measured person 2 feels one -point stimulation or two- points stimulation, inspecting two-points discrimination proficiency.

In the embodiment, as shown in Figs. 8 (A) and (B), two pulse- like voltages of which phases are shifted each other by approximately 180, are applied to two electrode pairs E. Namely , for example, the voltage shown in Fig..8(A) and the other voltage shown in Fig. 8(B) are applied to the electrode pairs E(n) and E(n+3) respectively.

Further, in the embodiment, first, the stimulation current is applied to the measured person 2 from the standard electrode pair E(n), measuring the minimum- perception current. Next, voltages are applied to two electrode pairs E so as to apply the stimulation currents of the measured minimum- perception current values to the measured person 2.

Fig. 9 is a flow chart showing a procedure of inspecting the two-points discrimination proficiency using the electrode sheet 33 as an example. First, the measured person 2 wears the electrode pair 3 (step S31.) Next, a voltage is applied to the electrode pair E(n) as a standard that the current value of the stimulation current given to the measured person 2 is gradually increased from "0" (step S32). Next, the measured person 2 pushes the stop switch of the manipulating button 5 held with his or her hand if he or she initially feels the stimulation current. Pushing the stop switch makes MPU 9 memorize the current value at that time as the minimum- perception current (step S32).

Then, voltages are applied to two electrode pairs E such as E(n) and E(n+1) (the initial value of "i" is three in the embodiment) (step S34.) More specifically, voltages are applied to two electrode pairs E so that the current values of the stimulation currents applied to the measured person 2 gradually increases from "0" and finally reach the minimum-perception current measured at step S33. Further, step S34 inspects whether the measured person feels one- point stimulation or two- points stimulation about the stimulation currents from the electrode pairs E(n) and E(n+i).

More specifically, first, an voltage is applied to two electrode pairs E such as E(n) and E(n+3) so as to inspect whether the measured person 2 feels one- point stimulation or two -points stimulation about the stimulation currents given by the electrode pairs E(n) and E(n+3). Second, voltages are applied to two electrode pairs E such as E(n) and E(n+2) so as to inspect whether the measured person 2 feels one- point stimulation or two -points stimulation about the stimulation currents given by the electrode pairs E(n) and E(n+2). Third, voltages are applied to two electrode pairs E such as E(n) and E(n+1) so as to inspect whether the measured person 2 feels one-point stimulation or two-points stimulation about the stimulation currents given by the electrode pairs E(n) and E(n+1).(Steps S34 to S36 implement these processes.) Fourth, a voltage is applied to two electrode pairs E with respect to the above three combinations, finally completing the inspection of two-points discrimination proficiency.

Here, these processes among steps S32 to S36 are controlled by MPU9 and others. But, the measured person 2 or a medical doctor may apply voltages the two electrode pairs E of the above three combinations in series.

In the embodiment, steps S32 and S33 is a step of measuring the perception- threshold value for standard electrode pair which is the minimum- perception current value by applying a voltage to the electrode pair E(n) as a standard in order to gradually increase the stimulation current. Further, steps S34 to S36 is a step of applying voltages to the electrode pairs E(n+3) to E(n+1) in series and a way of approaching to the electrode pair E(n) while also applying a voltage to the electrode pair E(n) as a standard in order to give the minimum- perception current value. The electrode pairs are arranged in a manner that the electrode pair E(n+3) is located at a position of the most far from the electrode pair E(n) as a standard and other electrode pairs are aligned toward the electrode pair E(n).

Accordingly, in the embodiment, voltages are applied to the electrode pairs E(n+3) to E(n+1) in series and a way of approaching to the electrode pair E(n) while a voltage is also applied to the electrode pair E(n) as a standard. The electrode pairs are arranged in a manner that the electrode pair E (n+3) is located at a position of the most far from the electrode pair E(n) as a standard and other electrode pairs are aligned toward the electrode pair E(n). Hence, these processes can inspect two-points discrimination proficiency in a manner that the measured person wears the electrode sheet 33 only one time without wearing it again. As a result, time of inspecting two-points discrimination proficiency can be shortened and the processes for it can be simplified.

Further, in the embodiment, step S34 applies voltages to two electrode pairs E so as to apply the stimulation current of the minimum- perception current values to the measured person 2 measured by step S33. Accordingly, these processes can objectively inspect two-points discrimination proficiency while making the minimum-perception current values as a standard. Further, nerves of the measured person 2 are not excessively stimulated since the stimulation currents of the minimum- perception current values flow in the person. As the result, two-points discrimination proficiency can be accurately inspected.

In the embodiment, step S34 applies two pulse -like voltages of which phases are shifted each other by approximately 180 °, to two electrode pairs E. Hence, the above process avoids simultaneous application of the stimulation currents to the measured person 2 from both the electrode pair E(n) as a standard and other electrode pairs E(n+1) to E(n+3). Accordingly , this process prevents the stimulation current applied to the measured person 2 from the standard electrode pair E(n) from interfering with the stimulation currents applied to the measured person 2 from the electrode pairs E(n+1) to E(n+3). As the result, two-points discrimination proficiency can be appropriately inspected.

Here, the current values of the stimulation currents given by the two electrode pairs E may not be the minimum- perception current values in step S34. Namely, voltages may be applied to the two electrode pairs E so that the current values of the stimulation currents given by the two electrode pairs E may be more than the minimum- perception current values in step S34.

Further, voltages may not be applied to the electrode pairs E such as E(n) and E(n+3) while the electrode pair E(n) is a standard electrode pair. First, voltages may be applied to the two electrode pairs E(n) and E(n+2) instead. Second, voltages may be applied to the two electrode pairs E(n) and E(n+1). Even in this case, two-points discrimination proficiency can be inspected. Namely, it is possible to inspect two-points discrimination proficiency if voltage are applied to any of the plurality of electrode pairs E in series and a way of approaching to the standard electrode pair. The plurality of electrode pairs E are arranged in a manner that any of the electrode pairs be located at a position far from the standard electrode pair by at least two or more electrode pairs and others of the electrode pairs be arranged in series and way of approaching the standard electrode pair.

Further, first, voltages may be applied to the two electrode pairs E(n) and E(n+3) while the electrode pair E(n) is a standard electrode pair. Second, voltages may be applied to the two electrode pairs E(n) and E(n+2). Voltages may not be applied to the two electrode pairs E such as E(n) and E(n+1) at that time.

A standard electrode pair may not be limited to the electrode pair E(n), but any of the electrode pairs E(n+1) to E(n+3) instead. In this case, step S32 applies a voltage to one of the electrode pairs E as a standard electrode pair, measuring the minimum-perception current. If the electrode pair E(n+3) is a standard electrode pair, voltages are applied to the two electrode pairs E in series as a way of combination of the electrode pair E(n+3) with other electrode pairs E(n) to E(n+2). If the electrode pair E(n+2) is a standard electrode pair, step 34 applies voltages to the two electrode pairs E in series as a way of combination of the electrode pair E(n+2) with other electrode pairs E(n) to E(n+1). If the electrode pair E(n+1) is a standard electrode pair, step 34 applies voltages to the two electrode pairs E in series as a way of combination of the electrode pair E(n+2) with other electrode pairs E(n+2) to E(n+3).

Here, in the embodiment, if four electrode pairs E are arranged in the electrode sheet 33, any one of the electrode pairs E can be a standard electrode pair. But in case of arranging three electrode pairs E in the electrode sheet 33, two-points discrimination proficiency cannot be inspected if one of the electrode pairs E located at the central position is a standard electrode pair. Namely, a standard electrode pair must be the electrode pair located at the position far from the end of the plurality of electrode pairs E arranged on the electrode sheet 33 by at least two electrode pairs.

Further, in case of inspecting two-points discrimination proficiency using the electrode sheet 33, first, voltages may be applied to the two electrode pairs E(n) and E(n+1) while the electrode pair E(n) is a standard electrode pair. Second, voltages may be applied to the two electrode pairs E(n) and E(n+1). Finally, voltages may be applied to the two electrode pairs E(n) and E(n+3). Namely, a voltage may be applied to the electrode pair E(n) as a standard while voltages may be applied to the electrode pairs E(n+1) to E(n+3) in series and a way of stepping away from the electrode pair E(n) and approaching to the electrode pair E(n+3) located at the position being the most far from the electrode pair E(n). Here the electrode pair E(n+1) is adjacent to the electrode pair E(n).

### Electrode Sheet Structure and Procedure Used for Inspecting Neural State of Skin Perceptual Band

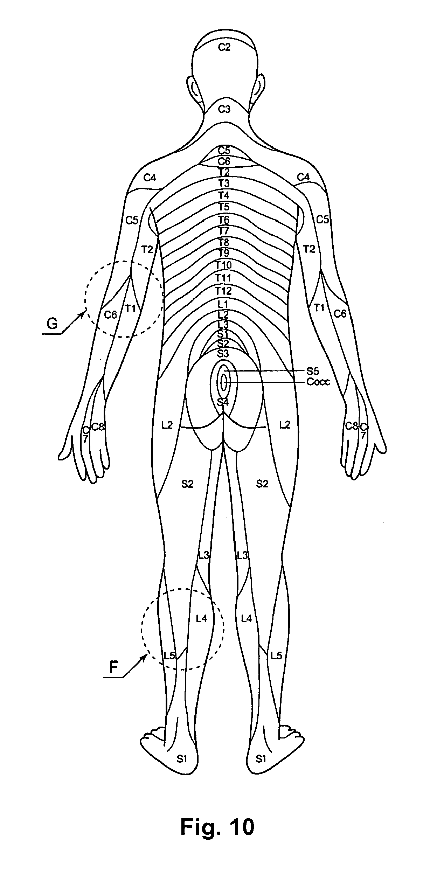

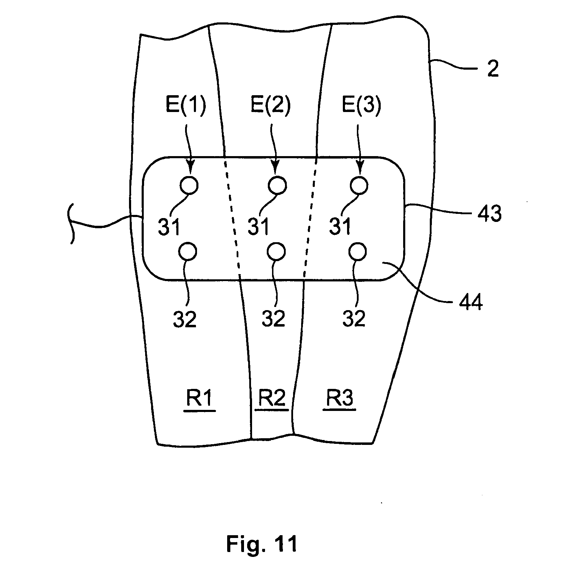

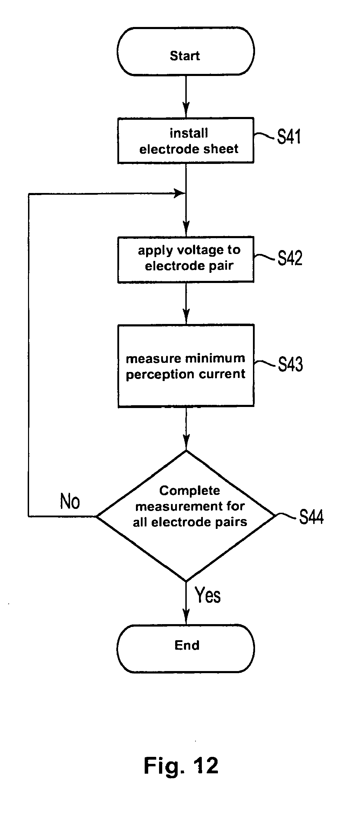

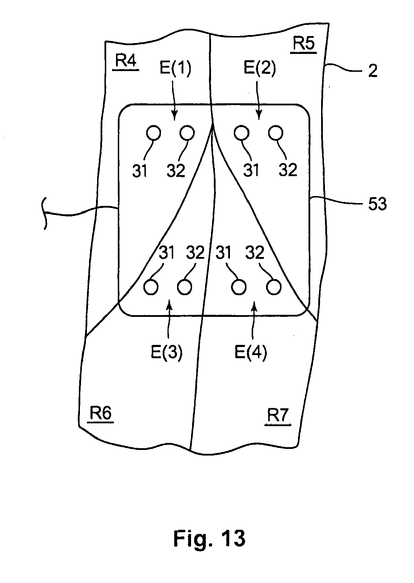

Fig. 10 is a diagram showing a skin perceptual band. Fig. 11 is a diagram showing a state of the measured person 2 who wears the electrode sheet 43 regarding the second embodiment of the invention. Fig. 12 is a flow chart showing a part of procedures measuring a minimum- perception current value in a plurality of regions of the skin perceptual band with using the electrode sheet shown Fig. 11. Fig. 13 is a diagram showing a state of the measured person 2 who wears the electrode sheet 53 regarding the second embodiment of the invention.

As shown in Fig. 10, the surface of a skin is classified into specific regions called as dermatome (a skin perceptual band). Each of regions is occupied by sensory nerve fibers of a spinal nerve root and the sensory nerve fibers transmit sensory information in each of regions to a specific spinal nerve root.

It is possible to inspect a neural state of plural regions of the skin perceptual band by using the stimulation-current providing device 1 of the embodiment. For example, it is possible to map the distribution of the minimum- perception currents in plural regions of a skin perceptual band by measuring the minimum- perception current in plural regions of a skin perceptual band. When a neural state of plural regions of the skin perceptual band is inspected by using the stimulation-current providing device 1, the electrode sheet 43 shown in Fig. 11 is installed on a F portion (a part of a leg of the measured person 2) in Fig. 10.

The electrode sheet 43 comprises a plurality of electrode pairs E ,each including a pair of the negative and positive electrodes 31 and 32 and a base sheet 44 fixing the plurality of electrode pairs E. In an example of Fig. 11, the electrode sheet 43 is provided with three electrode pairs E (1) to E(3). The base sheet 44 has an approximately rectangular shape.

The electrode pairs E(1) to E(3) are adjacently arranged each other in order with a predetermined interval from the one end to the other end of the electrode sheet 43. In the embodiment, as shown in Fig. 11, three electrode pairs E(1) to E(3) are arranged on the electrode sheet 43 so that they are aligned in regions R1 to R3 of a skin perceptual band of the measured person 2 respectively. Namely, the electrode pairs E(1) to E(3) are arranged on the electrode sheet 43 so that the negative and positive electrodes 31 and 32 of the electrode pair E(1) are aligned in the region R1, the negative and positive electrodes 31 and 32 of the electrode pair E(2) are aligned in the region R2 and the negative and positive electrodes 31 and 32 of the electrode pair E(3) are aligned in the region R3.

The electrode sheet 43 is connected to the body 4 shown in Fig. 1 and the like. More specifically, each of the electrode pairs E is connected to the voltage application unit comprising the boosting transformer 10, the voltage control circuit 11 and the output control circuit 12 via the protection circuit 13 and the current detection circuit 14. In the embodiment, it is possible for the voltage application unit to apply a voltage to arbitrary one of the electrode pairs E so as to give the stimulation current to the measured person 2.

In case of mapping the distribution of the minimum- perception current values in plural regions of a skin perceptual band using the electrode sheet 43 , voltages are applied to the electrode pairs E(1) to E(3) in series. At this time, voltages may be applied to the electrode pairs E(1) to E(3) in any orders. Further, the minimum- perception current values are measured in series at the time when the electrode pairs E(1) to E(3) give the stimulation currents to the measured person 2.

Fig. 12 shows a procedure of measuring the minimum-perception current in plural regions of a skin perceptual band using the electrode sheet 43 as an example. Namely, the electrode sheet 43 is installed on the measured person 2 so that the negative and positive electrodes of each of electrode pairs E(1) to E(3) are aligned to each of regions R1 to R3 (the negative and positive electrodes of each of electrode pairs E(1) to E(3) do not overlap more than two regions) (step S41).

Next, a voltage is applied to any of the electrode pairs E(1) to E(3) so that the current value of the stimulation current given to the measured person 2 is gradually increased from "0" (step S42). Next, the measured person 2 pushes the stop switch of the manipulating button 5 held with his or her hand if he or she initially feels the stimulation current. Pushing the stop switch makes MPU 9 memorize the current value at that time as the minimum- perception current (step S43).

Then, voltages are applied to other electrode pairs E in series and the minimum-perception currents are measured until completing minimum-perception currents for all electrode pairs E (steps S42 to S44). If minimum-perception current values for all electrode pairs E are completely measured, the measurement of minimum-perception current values in plural regions of a skin perceptual band is finished.

Here, all procedures from steps S42 to S44 are controlled by MPU9 and the like. But, the measured person 2 or a medical doctor may operate the stimulation-current providing device 1 so as to apply voltages to electrode pairs E(1) to E(3) in series.

In the embodiment, steps S42 and 44 are a step of applying voltages to the electrode pairs E(1) to E(3) arranged without overlapping two or more regions of a skin perceptual band of the measured person 2 so as to provide the stimulation currents for the measured person 2.

In the embodiment, electrode pairs E(1) to E(3) are arranged on the electrode sheet 43 so that a pair of the negative and positive electrodes 31 and 32 for each of electrode pairs are aligned in each of the regions R1 to R3 (without overlapping two or more regions of a skin perceptual band of het measured person 2.) Namely, it is possible to measure neural situations in plural regions of a skin perceptual band while the measured person wears the electrode sheet 43 only one time without doing it again. For example, the minimum- perception current values in plural regions of a skin perceptual band can be measured. Accordingly, time of inspecting neural situations in plural regions of a skin perceptual band can be shortened and the inspection can be simplified. Further, sensory nerve fibers corresponding to each of regions can be simply evaluated and a region of damaged spinal cord can be simply evaluated as the result of it.

The numbers of electrode pairs E are not limited to three, may be any numbers equal to or more than two. Further, the location of installing the electrode sheet is not limited to the F portion in Fig. 10. For example, as shown in Fig. 13, an electrode sheet 53 including four electrode pairs E(1) to E(4) may be installed on a G portion (an arm of the measured person 2.) In this case, four electrode pairs E(1) to E(4) are arranged on the electrode sheet 53 so that a pair of the negative and positive electrodes 31 and 32 for each of electrode pairs are aligned in each of regions R4 to R7 . In case of measuring the minimum- perception current value, a pair of the negative and positive electrodes 31 and 32 for each of four electrode pairs E(1) to E(4) are aligned in each of regions R4 to R7 of a skin perceptual band of the measured person 2. Namely, a pair of the negative and positive electrodes 31 and 32 for each of electrode pairs E(1) to E(4) is aligned not so as to overlap two or more regions of a skin perceptual band.

Further, at least two electrode pairs E may be arranged on the electrode sheet 43 so that a pair of the negative and positive electrodes 31 and 32 for each of electrode pairs is able to be aligned in each of the regions of a skin perceptual band. All electrode pairs E may not be necessarily arranged on the electrode sheet 43 so that a pair of the negative and positive electrodes 31 and 32 for each of electrode pairs is able to be aligned in each of the regions of a skin perceptual band. For example, the electrode pairs E(1) to E(3) may be arranged on the electrode sheet 43 so that the negative and positive electrodes 31 and 32 of the electrode pair E(1) are able to be aligned in the region R1, the negative and positive electrodes 31 and 32 of the electrode pair E(3) be aligned in the region R3 and the negative and positive electrodes 31 and 32 of the electrode pair E(2) be aligned with overlapping the regions R1 and R2 .

### Modification of Embodiment 2

In the above embodiments, when the stimulation is given to two points Y and Z of the surface of a body of the measured person 2, two-points discrimination proficiency is inspected by checking whether the measured person 2 feels one-point stimulation or two-points stimulation (see Fig. 7). Otherwise, for example, two-points discrimination proficiency may be inspected by the following; using an electrode sheet including two electrode pairs E(10) and E(11), voltages may be applied to two electrode pairs E(10) and E(11) so as to alternatively apply the stimulation currents from the two electrode pairs E(10) and E(11) arranged with a predetermined interval. Then, it may be measured whether the measured person feels one-point stimulation or two-points stimulation by alternatively receiving the stimulation currents. Here, in this case, two electrode pairs E (10) and E (11) are arranged with a predetermined space not so as to receive an influence of a voltage applied to these electrode pairs each other.

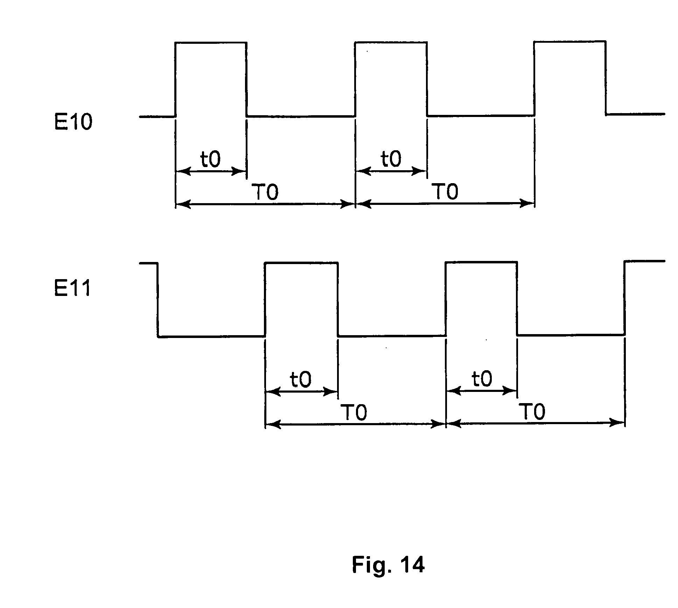

More particularly, two- points discrimination proficiency may be inspected by the following; pulse-like voltages, each having a predetermined switching period T0, may be applied to two electrode pairs E10 and E11 in a manner that on time "to" for voltages applied to E10 and E11 is shifted each other (see Fig. 14) while the switching period T0 is changed (namely, the frequency of the on time "to" is changed). The boundary between the switching period T0(or frequency) where the measured person feels one-point stimulation by receiving the stimulation current from the two electrode pairs E10 and E11, and the switching period T0(or frequency) where the measured person feels two-points stimulation by receiving the stimulation currents, may be measured. The inspected value corresponding to neural transmission speed may be obtained based on the above measured frequency boundary.

Further specifically, two-points discrimination proficiency may be inspected by the following: First, the minimum-perception current value is measured (a step for measuring the threshold value) by applying pulse -like voltages, each having the predetermined switching period T0, to any of the electrode pairs E10 and E11 so as to gradually increase the stimulation currents. Next, pulse -like voltages are applied to the electrode pairs E10 and E11 (a voltage application step) in a manner that the switching period T0 of the voltages gradually becomes longer than the initial switching period T0 (the frequency of the on time "t0" gradually becomes lower) and the on time "t0" for electrode pairs E10 and E11 is shifted each other as shown in Fig. 14 (for example , the phases of pulse -like voltages applied to electrode pairs E10 and E11 are shifted by 180 °). Further, the voltage application step applies voltages to the electrode pairs E10 and E11 so as to give the stimulation currents of the minimum-perception currents to the measured person 2 from the electrode pair 3. Here, the initial switching period T0 is the switching period T0 where the measured person 2 feels one-point stimulation based on the stimulation currents given from the two electrode pairs E10 and E11.

Then, voltages are applied to the electrode pairs E10 and E11 with gradually extending the switching period T0. When the measured person 2 who has felt one- point stimulation based on the stimulation currents feels two- points stimulation base on the stimulation currents, the switching period T0 (or the frequency of the on time "t0") at the time is recorded in MPU9. Further, the inspection value corresponding to neural transmission speed is obtained based on the recorded the switching period T0. The inspection regarding two-points discrimination proficiency is completed.

Accordingly, in the modification 2 of embodiments, pulse-like voltages are applied to the electrode pairs E10 and E11 with gradually extending the switching period T0. This application of the voltages can inspect whether the measured person 2 feels one- point stimulation about the stimulation currents from the electrodes E10 and E11 within a specific range among the switching period T0 or the measured person 2 feels two-points stimulation about the stimulation currents from the electrodes E10 and E11 within other specific range among the switching period T0. Hence, these processes can inspect two-points discrimination proficiency in a manner that the measured person wears the electrode sheet 33 only one time without wearing it again. As the result, time for inspecting two-points discrimination proficiency can be shortened. Further, inspecting two-points discrimination proficiency can be simplified.

Otherwise, two-points discrimination proficiency may be inspected by the following; by using an electrode sheet including three or more electrode pairs, it may be measured whether the measured person 2 feels one-point stimulation about the stimulation currents from the plural electrode pairs E with a specific range among the switching period T0 and/or t the measured person 2 feels plural- points stimulation about the stimulation currents from the plural electrode pairs E with other specific range among the switching period T0. Further, a voltage application step may apply voltages to the electrode pairs E10 and E11 while gradually shortening the switching period T0. In this case, the initial switching period T0 for measuring the minimum- perception current is the switching period T0 when the measured person 2 feels two-points stimulation based on the stimulation currents given from the two electrode pairs E10 and E11.

### Other Modification of Embodiment 2

In the above embodiment, the electrode sheets 33, 43 and 53 are installed on a leg or an arm of the measured person 3. Otherwise, these electrode sheets 33, 43 and 53 may be installed on a chin of the measured person 2. Namely, the stimulation-current providing device 1 of the embodiment including these electrode sheets 33, 43 and 53 can also be applied to the field of oral surgery.

## Claims

1. A stimulation-current providing device comprising:
- an electrode pair including a pair of positive and negative electrodes installed on a person to be measured;
- a voltage application unit that applies a voltage to the electrode pair so as to apply a stimulate current to the measured person from the electrode pair, wherein
the voltage application unit applies a pulse-like voltage having a predetermined switching period to the electrode pair and applies the stimulation current to the measured person while gradually extending or shortening the switching period.

2. A stimulation-current providing device comprising:
- an electrode sheet that includes a plurality of electrode pairs and is installed on a measured person, each of the pair electrodes being composed of a pair of negative and positive electrodes: and
- a voltage application unit that applies voltages to the electrode pairs so as to apply a stimulation current to the measured person from the electrode pairs, wherein
the voltage application unit provides the stimulation current for the measured person by applying pulse-like voltages that have a predetermined switching period to a plurality of the electrode pairs in a manner of shifting "on time" of the voltages applied to the plural electrode pairs each other and applying the voltage to the plural electrode pairs with gradually extending or shortening the switching period.

3. A stimulation-current providing device comprising:
- an electrode sheet that includes a plurality of electrode pairs and is installed on a measured person , each of the electrode pairs including a pair of negative and positive electrodes: and
- a voltage application unit that applies a voltage to the electrode pair so as to apply a stimulate current to the measured person from the electrode pair, wherein
the plurality of electrode pairs are arranged on the electric sheet so as to be aligned in order from a central nerve to a peripheral nerve of the measured person,
wherein the voltage application unit applies a voltage to every one of the plurality of electrode pairs from one end to the other end of the electrode -pairs arrangement so as to provide a stimulation current for the measured person.

4. A stimulation-current providing device comprising:
- an electrode sheet that includes a plurality of at least three or more electrode pairs and is installed on a measured person, each of the electrode pairs including a pair of negative and positive electrodes: and
- a voltage application unit that applies a voltage to the electrode pairs so as to apply a stimulate current to the measured person from the electrode pairs, wherein the plurality of electrode pairs are arranged in order on the electrode sheet,
wherein, if a specific electrode pair among a plurality of electrode pairs arranged on a electrode sheet is defined as a standard electrode pair, the standard electrode pair being placed at the location far from the end of the arranged plurality of electrode pairs by at least two electrode pairs, the voltage application unit applies a stimulate current to the measured person by the following; first, applying a voltage to the standard electrode pair, and ,then, applying a voltage to any of the plurality of electrode pairs in series and a way of approaching to the standard electrode pair or stepping away from the standard electrode pair while applying a voltage to the standard electrode pair, one pair of the plurality of electrode pairs being arranged in a manner that any of the electrode pairs be located at a position far from the standard electrode pair by at least two or more electrode pairs and others of the electrode pairs be arranged in series and way of approaching the standard electrode pair.

5. A stimulation-current providing device comprising:
- an electrode sheet that includes a plurality of electrode pairs and is installed on a measured person , each of the electrode pairs including a pair of negative and positive electrodes: and
- a voltage application unit that applies a voltage to the electrode pairs so as to apply a stimulate current to the measured person from the electrode pairs,
wherein the at least two or more electrode pairs are arranged on the electrode sheet so that the pair of the negative and positive electrodes are aligned without overlapping two or more regions of a skin perceptual band of the measured person.

6. A method of controlling a stimulation-current providing device that applies a voltage to an electrode pair including a pair of negative and positive electrodes and provides a stimulation current for a measured person from the electrode pair; the method comprising:
- measuring a minimum- perception current value by applying a pulse - like voltage to the electrode pair, the pulse -like voltage having a predetermined initial switching period in order to gradually increase the stimulation current, and the minimum- perception current value being a current value of the stimulation current at the time when the measured person feels initial stimulation (a step of measuring a threshold value); and
- applying a pulse-like voltage to the electrode pair while gradually extending or shortening the switching period, compared to an initial switching period (a voltage application step).

7. A method of controlling a stimulation-current providing device that includes a plurality of electrode pairs, each having a pair of positive and negative electrodes and being arranged on a electrode sheet , and applies a voltage to the electrode pairs in order to provide a stimulation current for a measured person, the method comprises;
- measuring a minimum-perception current value by applying a pulse-like voltage to any of the plurality of electrode pairs, the pulse-like voltage having a predetermined initial switching period in order to gradually increase the stimulation current, and the minimum-perception current value being a current value of the stimulation current at the time when the measured person feels initial stimulation (a step of measuring a threshold value); and
- applying the pulse- like voltage to the plurality of electrode pairs, while gradually extending or shortening the switching period, compared to an initial switching period and shifting timing (on time) of the voltage applied to the plurality of pair electrodes each other (a voltage application step).

8. A method of controlling a stimulation-current providing device comprising a step of providing a stimulation current for a measured person from a plurality of electrode pairs by applying a voltage to the plurality of electrode pairs in series and a way of approaching to one end of electrode -pairs arrangement from the other end of the electrode -pairs arrangement, each of the electrode pairs including a pair of positive and negative electrodes, and being arranged on an electrode sheet so as to be aligned in order from a central nerve to a peripheral nerve of the measured person.

9. A method of controlling a stimulation-current providing device that includes at least three or more pluralities of electrode pairs, each composed of a pair of positive and negative electrodes and arranged on a electrode sheet in order, and applies a voltage to the plurality of the electrode pairs so as to provides a stimulation current for a measured person, the method comprising:
- defining a specific electrode pair among the plurality of electrode pairs arranged on the electrode sheet as a standard electrode pair, the standard electrode pair being placed at the location far from the end of the arranged plurality of electrode pairs by at least two or more electrode pairs,
- measuring a minimum- perception current value that is the stimulation current value at the time when the measured person feels initial stimulation by applying a voltage to the standard electrode pair so as to gradually increase the stimulation current, (a step of measuring a perception threshold value for a standard electrode pair).
- applying a voltage to the standard electrode pair so as to provide a stimulation current as the minimum-perception current value after the step of measuring the perception threshold value for standard electrode pair, while applying a voltage to any of the plurality of electrode pairs in series and a way of approaching to the standard electrode pair or stepping away from the standard electrode pair, one pair of the plurality of electrode pairs being arranged in a manner that any of the electrode pairs be located at a position far from the standard electrode pair by at least two or more electrode pairs and others of the electrode pairs be arranged in series and way of approaching the standard electrode pair (a voltage application step).

10. The method of controlling a stimulation-current providing device according to claim 9, wherein the voltage applied to the electrode pair is a pulse-like voltage in the voltage application step; and a phase of the voltage, applied to the standard electrode pair, is shifted from a phase of the voltage ,applied to the plurality of electrode pairs being arranged in the order from the electrode pair located at a position far from the standard electrode pair by at least two or more electrode pairs toward the standard electrode pair, by approximately 180 °.

11. A method of controlling a stimulation-current providing device that includes a plurality of electrode pairs, composed of a pairs of positive and negative electrodes and arranged on a electrode sheet, and applies a voltage to the plurality of electrode pairs so as to provides a stimulation current for a measured person, the method comprises a step of providing a stimulation current for the measured person by applying a voltage to the at least two electrode pairs in order, these electrode pairs including a pair of negative and positive electrodes that are aligned without overlapping two or more regions of a skin perceptual band of the measured person.
